(19) **Europäisches Patentamt / European Patent Office / Office européen des brevets**

(11) **EP 2 002 788 A1**

(12) **EUROPEAN PATENT APPLICATION**

(43) Date of publication:
**17.12.2008 Bulletin 2008/51**

(51) Int Cl.:
**A61B 5/20** (2006.01)

(21) Application number: **07110276.8**

(22) Date of filing: **14.06.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Stichting voor de Technische Wetenschappen**
**3527 JP  Utrecht (NL)**

(72) Inventors:
• **Idzenga, Tim**
  **4811 NB Breda (NL)**
• **Pel, Johannes Jacob Mient**
  **2951 AL Alblasserdam (NL)**
• **van Mastrigt, Robert**
  **3281 KD Numansdorp (NL)**

(74) Representative: **van Loon, C.J.J.**
**Vereenigde**
**Johan de Wittlaan 7**
**2517 JR Den Haag (NL)**

(54) **A method, a computer program and an apparatus for quantification of a degree of obstruction in a liquid passageway.**

(57)    The invention relates to a method for quantification of a degree of obstruction in a liquid passageway, notably in a male urethra. Data related to sonic waves generated by a liquid passing the obstruction may be accessed from a suitable file, or, alternatively may be directly obtained as a result of a measuring step 2. For determining the degree of obstruction of the urethra the measuring step 2 may be performed by arranging at least one sensor, notably a microphone, in the area of the perineum for detecting sonic waves generated by a liquid (urine) passing the obstruction. At the step 5 the power spectrum of the obtained data related to the sonic waves is calculated, after which a suitable feature 6a, 6b, or 6c representative of the power spectrum is selected. Preferable embodiments of the feature comprise a weighted average frequency of the power spectrum, a standard deviation of the power spectrum, or a skewness of the power spectrum, discussed above. The degree of obstruction may be quantified at step 7 by comparing the selected feature with a reference value 8 for the said feature. Preferably, the reference feature is obtained using a calibration experiment in a suitable phantom.

Figure 3

EP 2 002 788 A1

**Description**

FIELD OF THE INVENTION

The invention relates to a method for quantification of a degree of obstruction in a liquid passageway.

The invention further relates to a computer program for quantification of a degree of obstruction in a liquid passageway.

The invention still further relates to an apparatus for quantification of a degree of obstruction in a liquid passageway.

BACKGROUND OF THE INVENTION

Lower Urinary Tract Symptoms (LUTS) in ageing males, e.g. a weak stream, dribbling or frequent nightly voiding, mostly result from an obstructed urethra caused by an enlarged prostate (Benign Prostatic Enlargement or BPE) or a weakly contracting bladder. At present the authorative International Continence Society (ICS) recommends a provisional method for differentiating between these causes by diagnosing Bladder Outlet Obstruction (BOO). This method is based on the maximum urinary flow rate and the associated bladder (detrusor) pressure, graphically represented in a pressure-flow plot. The most frequently used method for measuring the detrusor pressure is inserting a catheter into the bladder via the urethra. This method is invasive, time-consuming and uncomfortable for the patient. Therefore, not all patients with LUTS are urodynamically tested. This may lead to erroneous diagnoses and failing treatment. Furthermore, the transurethral catheters used induce the risk of urinary tract infection and urethral trauma. Presently, more patient-friendly methods are being developed and validated to diagnose BOO in patients with LUTS. Most of these methods are based on non-invasive assessment of pressure and flow signals during manipulated/interrupted voiding or on long-term changes caused by BOO, such as changes in bladder-wall thickness.

It is a disadvantage of the known methods in that they require an interruption of the voiding process, notably a plurality of such interruptions. This complicates a measurement, which necessitates that medically qualified personnel are present to carry out the measurement. In addition, such interruptions are not comfortable for the patient and an increase in anxiety of the patient may change the voiding process which may lead to an inaccurate measurement result.

SUMMARY OF THE INVENTION

It is an object of the invention to provide a non-invasive method for quantification of the degree of urethral obstruction, having increased accuracy.

To this end the method according to the invention comprises the steps of:

- accessing data related to sonic waves generated by a liquid passing the liquid passageway;
- calculating a power spectrum of said data;
- determining at least one specific feature representative to the power spectrum;
- comparing the said at least one specific feature with at least one reference for quantification of the degree of obstruction.

The method according to the invention is based on the insight that aspects of the recorded sonic waves produced, for example by the urine passing through an obstruction of the urethra are representative to the degree of the obstruction with high reproducibility and accuracy. In particular, it has been found that the power spectrum calculated for the detected sonic waves comprises specific features which are directly related to the degree of obstruction. The power spectrum can be calculated using per se known Fast Fourier Transform. By comparing at least one specific feature with at least one reference, quantification of the degree of obstruction is obtained.

It is noted that the method according to the invention represents further insights of the inventors, compared to initial phantom experiments, published in an article of Idzenga et al "Variation of recorded noise with distance from an obstruction in a polyvinyl alcohol model of the urethra", ICS abstract 2005, online published at http://www.icsoffice.org/.

In this article, it is suggested that for a clinical application noise should be recorded at more than one location at the perineum. However, continuous research has led to a conclusion that although a shape of the power spectrum changes substantially with a distance from the obstruction, respective values of specific features of the power spectrum are substantially invariant to the distance to the obstruction. The latter enables the method according to the invention to be used in clinical practice yielding accurate and reliable results.

The sonic waves are suitably recorded by means of a microphone, notably a piezoceramic microphone, which is arranged near the liquid passageway downstream of the obstruction. For medical application, the microphone may be arranged at the perineum of a male. The sonic waves are then detected under condition of voiding of the male. It is noted that in this case the process of voiding can be conducted in an uninterrupted, patient-friendly manner.

In an embodiment of the method according to the invention a weighted average frequency $f_c$ of the power spectrum is selected for the said specific feature, it, for example, being given by a formula:

$$f_c = \frac{\int_{f_0}^{f_1} f \cdot P(f) \cdot df}{\int_{f_0}^{f_1} P(f) \cdot df},$$

wherein P(f) represents the power spectrum,
$f_0$ is a first boundary frequency,
$f_1$ is a second boundary frequency.

Preferably, the first boundary frequency $f_0$ is selected in a range of 300 - 500 Hz, preferably 400 Hz. It was found in a model that for the weighted average frequency $f_c$ of the power spectrum with $f_0$ set at or about 400 Hz a maximum predictable value for the degree of obstruction is obtained. The second boundary frequency may be set as high as 2500 Hz, preferably 1000 Hz.

In a further embodiment of the method according to the invention a standard deviation σ in the power spectrum is selected for the said specific feature, it, for example, being given by a formula:

$$\sigma = \sqrt{\frac{\int_{f_0}^{f_1} (f - f_c)^2 \cdot P(f) \cdot df}{\int_{f_0}^{f_1} P(f) \cdot df}},$$

wherein P(f) represents the power spectrum,
$f_0$ is a first boundary frequency,
$f_1$ is a second boundary frequency.

Preferably, in this case the first boundary frequency $f_0$ is selected in a range of 100 - 300 Hz, more preferably about 200 Hz. It is found that when the standard deviation σ in the power spectrum is selected, the maximum predictable value for the degree of obstruction is obtained for the first boundary frequency of about 200 Hz. The second boundary frequency may be set as high as 2500 Hz, preferably 1000 Hz.

In a further embodiment of the method according to the invention the skewness $\gamma_1$ of the power spectrum is selected for the said at least one feature, it, for example, being given by a formula:

$$\gamma_1 = \frac{\int_{f_0}^{f_1} (f - f_c)^3 \cdot P(f) \cdot df \Big/ \int_{f_0}^{f_1} P(f) \cdot df}{\left[ \int_{f_0}^{f_1} (f - f_c)^2 \cdot P(f) \cdot df \Big/ \int_{f_0}^{f_1} P(f) \cdot df \right]^{3/2}},$$

wherein P(f) represents the power spectrum,
$f_0$ is a first boundary frequency,
$f_1$ is a second boundary frequency.

Preferably, the first boundary frequency $f_0$ is selected in a range of 500 - 700 Hz, more preferably about 600 Hz. It is found that when the $\gamma_1$ of the power spectrum is selected, the maximum predictable value for the degree of obstruction is obtained for the first boundary frequency of about 600 Hz. The second boundary frequency may be set as high as 2500 Hz, preferably 1000 Hz.

It is noted that it has been found that all three features ($f_c$, σ, γ) are suitable for reliable quantification of a degree of obstruction in a liquid passageway in a living body, notably in the urethra, substantially independent of a position of the sensor detecting the sonic waves with respect to a position of the obstruction. It is further noted that also a statistical significance of each feature ($f_c$, σ, γ) was investigated. It was found that surprisingly the standard deviation σ yielded the most significant predictor of the degree of obstruction. With $f_0$ and $f_1$ set at 200 and 2500 Hz, respectively, the standard deviation of the power spectrum predicted 89% of the phantom measurements correctly.

In a further embodiment of the method according to the invention, data related to sonic waves generated by the liquid passing through the said liquid passageway comprises a plurality of respective sonic wave spectra detected for a corresponding plurality of measuring positions with respect to a position of the obstruction.

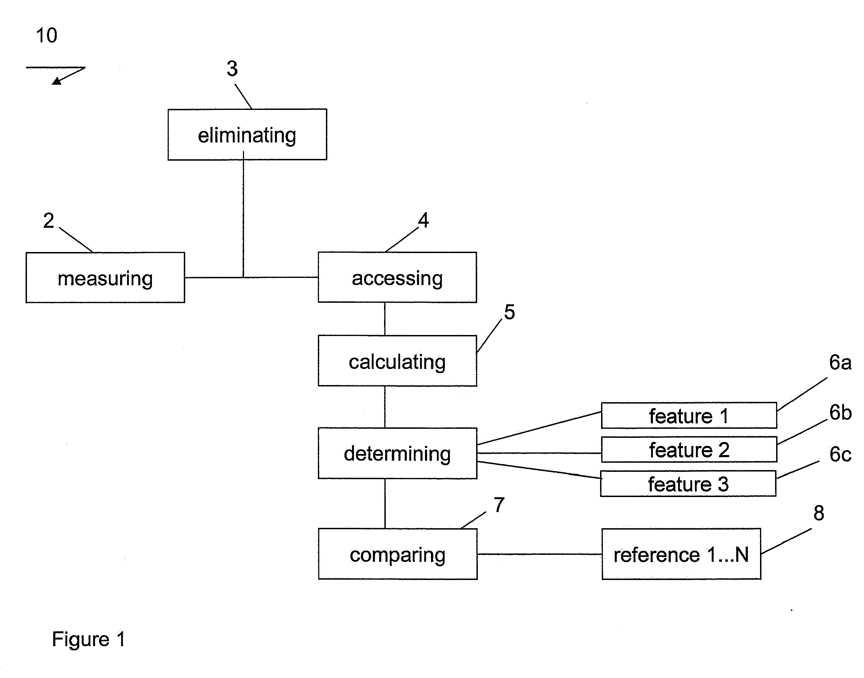

In accordance with this technical measure it is possible to eliminate the influence of the position of the sensor with respect to the obstruction on the accuracy of the result. Such elimination can be carried out by comparing respective sonic wave spectra measured by different sensors simultaneously for the same liquid flow. The method according to the invention will be discussed in further detail with reference to Figure 1.

The computer program according to the invention comprises instructions for causing a processor to carry out the steps of the method as is discussed with reference to the foregoing.

An apparatus according to the invention comprises:

- input for accessing data related to sonic waves generated by the liquid passing the said liquid passageway;
- a processor for analyzing the data, comprising

  i. calculating power spectrum for the said data;
  ii. determining at least one specific feature representative to the power spectrum;
  iii. comparing the said at least one specific feature with at least one reference for quantification of the degree of obstruction.

The apparatus according to the invention provides means for accurate determination of a degree of obstruction in a liquid passageway, notably male urethra. The data may be acquired earlier and may be suitably stored. It is also possible that the apparatus according to the invention comprises a remote computer arranged for processing data supplied by one or more remote clients. It is noted that the input may be arranged to use suitable per se known communication means for accessing the data, including telecommunication and wireless communication. Preferably, the apparatus according to the invention comprises at least one sensor for detecting the said sonic waves. In this case an intelligent, preferably, non-invasive instrument is provided for accurate determination of a degree of obstruction, notably in a human or animal liquid passageway. More preferably, the apparatus according to the invention comprises a plurality of sensors arranged to detect respective spectra of sonic waves simultaneously. Preferably, said sensors comprise suitable microphones, like piezoceramic microphones, for simultaneous non-invasive detection of respective sonic wave spectra at different locations with respect to the position of the obstruction. The processor of the apparatus may be accordingly arranged to process the acquired spectra substantially simultaneously for eliminating influence of a position of each respective microphone with regard to the obstruction. This feature improves accuracy of said quantification even further.

These and other aspects of the invention will be further discussed with reference to figures.

BRIEF DESCRIPTION

Figure 1 presents a schematic view of an embodiment of the method according to the invention.
Figure 2 presents in a schematic way characteristic dependencies of specific features on a degree of obstruction.
Figure 3 presents a schematic view of an embodiment of the apparatus according to the invention.

DETAILED DESCRIPTION

Figure 1 presents a schematic view of an embodiment of the method according to the invention. The method 10 according to the invention may commence at the step 4 of accessing data related to sonic waves generated by a liquid passing through a suitable liquid passageway. It is noted that although the method has been thoroughly validated in the clinical practice it is applicable to industrial fields as well, notably for analysis of suitable pipes. This may in particular be advantageous for investigating oil pipes. In the medical area, the method according to the invention is suitable for non-invasive determination of a degree of obstruction of the urethra in males, or a degree of obstruction of an artery, for example a carotid artery in males and females. For purposes of conciseness the method according to the invention will be discussed with reference to medical applications, it will be appreciated that industrial applications are conducted in a similar way.

Data related to sonic waves may be accessed from a suitable file, or, alternatively may be directly obtained as a result of a measuring step 2. For determining the degree of obstruction of the urethra the measuring step 2 may be performed by arranging at least one sensor, notably a microphone, in the area of the perineum for detecting sonic waves generated by a liquid (urine) passing the obstruction. Preferably, during the step 2 a plurality of sensors is arranged in the area of the perineum for eliminating a dependence of the sensor reading on a distance to the obstruction. For the latter, the method 10 according to the invention comprises the step 3 wherein respective data simultaneously obtained by the said plurality of sensors is normalized, compared or processed in another suitable way for purposes of said elimination. It is noted that the data may comprise raw data representing the sonic waves or may comprise suitably processed data, like filtered and/or noise reduced sonic waves.

It is noted that step 4 may be implemented substantially in real-time following the data acquisition of step 2. Alternatively, step 4 may be implemented at a suitable later moment, for example when data acquired for a plurality of patients or a plurality of data acquisition moment for the same patient is being analyzed. The latter option may be advantageous in case when measurement data are forwarded from different measurement cites to a common data analysis cite. This configuration may be advantageous for comparative studies, for example.

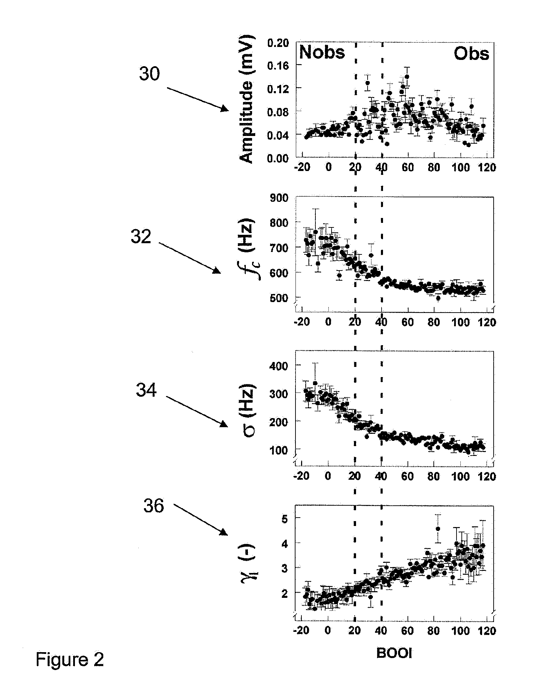

Subsequently, the method according to the invention follows to the step 5 of calculating the power spectrum of the obtained data related to the sonic waves. Preferably, the data signals representative of the sonic waves is used. The power spectrum is calculated using Fast Fourier Transform. The method according to the invention further comprises the step 6 of determining a suitable feature 6a, 6b, or 6c representative of the power spectrum. Preferable embodiments of the feature comprise a weighted average frequency of the power spectrum, a standard deviation of the power spectrum, or a skewness of the power spectrum, discussed above. These features are discussed in more detail with reference to Figure 2. It is noted that it is also possible to use more than one of the said features simultaneously for the purposes of determining the degree of obstruction with elevated accuracy. In addition, it is possible to use other features of the power spectrum to quantify the degree of obstruction.

In order to quantify a degree of obstruction, for example as a percentage of an initial area, the obtained feature is compared at step 7 with a reference value 8 for the said feature. Preferably, the reference feature is obtained using a calibration experiment in a suitable phantom.

Figure 2 presents in schematic way characteristic dependencies of specific features on a degree of obstruction. Figure 2 shows experimental results obtained for a number of aspects of the detected sonic waves. Feature 30 relates to average amplitude of the sonic waves. Feature 32 relates to a weighted average frequency in the power spectrum calculated for the detected sonic waves. Feature 34 relates to a standard deviation of the power spectrum calculated for the detected sonic waves. Feature 36 relates to a skewness of the power spectrum calculated for the detected sonic waves. It is seen from Figure 2 that features 32, 34, 36 demonstrate a monotonic dependence on the degree of obstruction BOOI, which renders them to be good means for providing quantitative assessment of the degree of obstruction in practice.

Although data shown in Figure 2 relate to laboratory data obtained for a simulation phantom, such measurements can be used for calibration purposes for calibrating the sensor to be used in clinical applications. The respective curves for the features 32, 34, 36 may be recorded as suitable reference data and can be used for comparison with clinically obtained data. Alternatively, it is possible to parameterize the curves and to store reference data as a suitable tabulation or function.

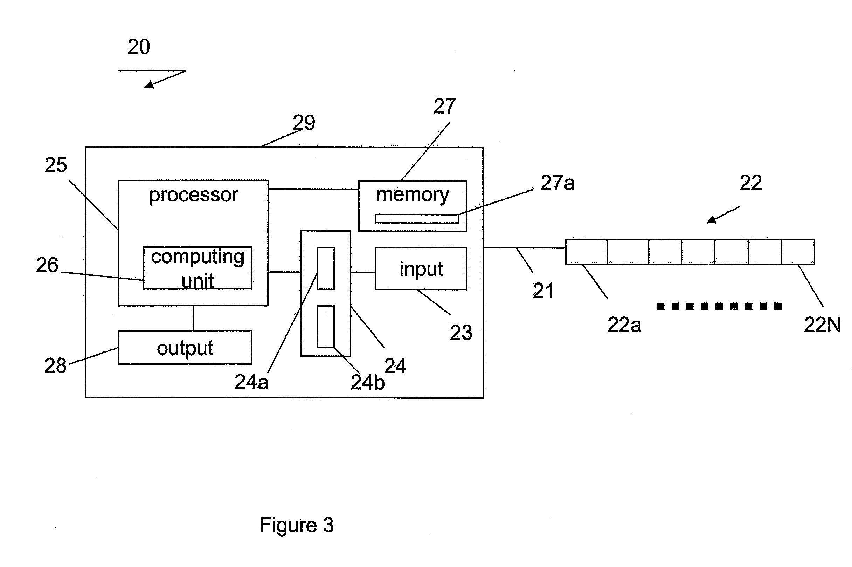

Figure 3 presents a schematic view of an embodiment of the apparatus according to the invention. The apparatus 20 comprises a housing 29 with electronics and a sensor 22, notably a microphone, for detecting data related to sonic waves generated by a liquid passing an obstruction in a liquid passageway. Preferably, the sensor 22 comprises an array 22a... 22N, or a suitable plurality of individual sensors which can be placed at a target area independently from each other. For the multiple sensor arrangement the cabling 21 between the housing 29 and the sensor 22 comprises a suitable plurality of data lines.

The housing 29 comprises an input 23 which can be arranged to collect data from the sensor 22, or to access data, for example from a file. Input 23 may also be arranged to access data in a wireless mode. The housing 29 may further comprise a suitable data processing unit 24, which may comprise suitable amplifiers 24a, suitable filters 24b and further suitable electronics. The data is then analyzed by the processor 25 comprising a computing unit 26 arranged for calculating the power spectrum for the said data, determining at least one of the selected features representative to the power spectrum and for comparing the said at least one of the specific features with at least one reference for quantification of the degree of obstruction. The reference value or values may be stored in a memory unit 27 of the apparatus 20 according to the invention. The output value of the degree of obstruction is put out via the output means 28, notably a display. Preferably, the obtained value of the degree of obstruction is also stored in the memory unit 27 in an appropriate file.

The apparatus 20 according to the invention may be operated by a suitable computer program product arranged for causing the processor 25 to carry out the steps of the method as is discussed with respect to the foregoing. The computer program 27a comprises an instruction for causing the processor 25 to access data related to sonic waves generated by a liquid passing through a suitable liquid passageway. Data related to sonic waves may be accessed from a suitable file, or, alternatively may be directly obtained as a result of a measuring step using sensor or sensors 22. In case a plurality of sensors is used for the measurement, the computer program 27a may comprise a further instruction to suitably process, notably normalize, measured data for eliminating any positional influence on the accuracy of data interpretation.

The computer program 27a further comprises an instruction for causing the computing unit 26 to derive the power spectrum for the obtained data related to the sonic waves. The power spectrum is calculated using Fast Fourier Transform, the transformation algorithm may be stored in the memory unit 27 as a separate subroutine. The computer program 27a further comprises an instruction for causing the computing unit 26 to determine a suitable feature representative of the power spectrum. Preferable embodiments of the feature comprise a weighted average frequency, a standard deviation of the power spectrum, or a skewness of the power spectrum, discussed above. In order to quantify the degree of obstruction, for example as a percentage of an initial area, the computer program 27a further comprises an instruction for causing the computing unit 26 to compare the obtained feature with a reference feature, notably being stored in a suitable accessible file in the memory unit 27.

While specific embodiments have been described above, it will be appreciated that the invention may be practiced otherwise than as described. The descriptions above are intended to be illustrative, not limiting. Thus, it will be apparent to one skilled in the art that modifications may be made to the invention as described in the foregoing without departing from the scope of the claims set out below.

**Claims**

1.  A method for quantification of a degree of obstruction in a liquid passageway comprising the steps of:

    - accessing data related to sonic waves generated by a liquid passing the liquid passageway;
    - calculating a power spectrum of said data;
    - determining at least one specific feature representative of the power spectrum;
    - comparing the said at least one specific feature with at least one reference for quantification of the degree of obstruction.

2.  A method according to claim 1, wherein a weighted average frequency $f_c$, of the power spectrum is selected for the said at least one feature, preferably being given by a formula:

$$f_c = \frac{\int_{f_0}^{f_1} f \cdot P(f) \cdot df}{\int_{f_0}^{f_1} P(f) \cdot df},$$

    wherein P(f) represents the power spectrum,
    $f_0$ is a first boundary frequency,
    $f_1$ is a second boundary frequency.

3.  A method according to claim 2, wherein $f_0$ is selected in a range of 300- 500 Hz, preferably 400 Hz.

4.  A method according to claim 1, wherein a standard deviation $\sigma$ in the power spectrum is selected for the said at least one feature, preferably being given by a formula:

$$\sigma = \sqrt{\frac{\int_{f_0}^{f_1}(f - f_c)^2 \cdot P(f) \cdot df}{\int_{f_0}^{f_1} P(f) \cdot df}} \, ,$$

wherein P(f) represents the power spectrum,
$f_0$ is a first boundary frequency,
$f_1$ is a second boundary frequency.

5. A method according to claim 4, wherein $f_0$ is selected in a range of 100 - 300 Hz, preferably 200 Hz.

6. A method according to claim 1, wherein a skewness $\gamma 1$ of the power spectrum is selected for the said at least one feature, preferably being given by a formula:

$$\gamma_1 = \frac{\left. \int_{f_0}^{f_1}(f - f_c)^3 \cdot P(f) \cdot df \middle/ \int_{f_0}^{f_1} P(f) \cdot df \right.}{\left[ \left. \int_{f_0}^{f_1}(f - f_c)^2 \cdot P(f) \cdot df \middle/ \int_{f_0}^{f_1} P(f) \cdot df \right. \right]^{3/2}} \, ,$$

wherein P(f) represents the power spectrum,
$f_0$ is a first boundary frequency,
$f_1$ is a second boundary frequency.

7. A method according to claim 6, wherein $f_0$ is selected in a range of 500 - 700 Hz, preferably 600 Hz.

8. A method according to any one of the preceding claims, wherein data related to sonic waves generated by the liquid passing through the said liquid passageway comprises a plurality of respective sonic wave spectra detected for a corresponding plurality of measuring positions with respect to a position of the obstruction.

9. A method according to any one of the preceding claims further comprising the step of:

   - detecting sonic waves generated by a liquid passing the said liquid passageway.

10. A method according to any one of the preceding claims, wherein the liquid passageway forms part of a living body, notably the urethra.

11. A computer program product comprising instructions for causing a processor to carry out the steps of the method according to any one of the preceding claims.

12. An apparatus for quantifying a degree of obstruction in a liquid passageway comprising:

   - input for accessing data related to sonic waves generated by the liquid passing the said liquid passageway;
   - a processor for analyzing the data, comprising

      i. calculating power spectrum for the said data;
      ii. determining at least one specific feature representative of the power spectrum;
      iii. comparing the said at least one specific feature with at least one reference for quantification of the degree of obstruction.

**13.** An apparatus according to claim 12, further comprising at least one sensor for detecting the said sonic waves.

**14.** An apparatus according to claim 13, comprising a plurality of sensors arranged to detect respective spectra of sonic waves simultaneously.

**15.** An apparatus according to claim 14, wherein the processor is further arranged to analyze the said plurality of respective spectra.

EP 2 002 788 A1

Figure 1

Figure 2

Figure 3

**European Patent Office**

## EUROPEAN SEARCH REPORT

Application Number

EP 07 11 0276

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | TIM IDZENGA ET AL: "Perineal noise recording as a non-invasive diagnostic method of urinary bladder outlet obstruction: A study in polyvinyl alcohol and silicone model urethras" NEUROUROLOGY AND URODYNAMICS, WILEY-LISS, US, vol. 24, no. 4, 2005, pages 381-388, XP009089656 ISSN: 0733-2467 * the whole document * ----- | 1,11-15 | INV. A61B5/20 |
| A | IDZENGA ET AL: "Variation in noise recorded distally to a urethral obstruction related to cross-sectional area and flow pattern" JOURNAL OF BIOMECHANICS, PERGAMON PRESS, NEW YORK, NY, US, vol. 39, 2006, page S635, XP005618426 ISSN: 0021-9290 * the whole document * ----- | 1-15 | |
| | | | TECHNICAL FIELDS SEARCHED (IPC) |
| A | US 3 363 619 A (KEITZER WALTER A) 16 January 1968 (1968-01-16) * column 1 - column 5 * ----- | 1-15 | A61B |

The present search report has been drawn up for all claims

| Place of search | Date of completion of the search | Examiner |
|---|---|---|
| Munich | 20 September 2007 | Rivera Pons, Carlos |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 07 11 0276

This annex lists the patent family members relating to the patent documents cited in the above-mentioned European search report.
The members are as contained in the European Patent Office EDP file on
The European Patent Office is in no way liable for these particulars which are merely given for the purpose of information.

20-09-2007

| Patent document cited in search report | | Publication date | Patent family member(s) | | Publication date |
|---|---|---|---|---|---|
| US 3363619 | A | 16-01-1968 | FR | 1472772 A | 10-03-1967 |